# EUROPÄISCHE PATENTSCHRIFT

(11) **EP 1 281 704 B1**
(45) Veröffentlichungstag und Bekanntmachung des Hinweises auf die Patenterteilung: **23.01.2008**
(21) Anmeldenummer: 02016707.8
(22) Anmeldetag: 26.07.2002
(51) Int. Cl.: C07D 263/42

(54) **Verfahren zur kontinuierlichen Herstellung von substituierten Oxazolen**
Process for the the preparation of substituted oxazoles in a continous manner
Procedé pour la préparation d'oxazoles en continu

(30) Priorität: 03.08.2001 DE 10137627; 05.03.2002 DE 10209447
(43) Veröffentlichungstag der Anmeldung: 05.02.2003
(73) Patentinhaber: BASF Aktiengesellschaft, 67056 Ludwigshafen (DE)
(72) Erfinder: Rust, Harald, 67435 Neustadt (DE); Burkart, Kirsten, 67069 Ludwigshafen (DE); Faust, Tillmann, Dr., 67256 Weisenheim (DE); Henkelmann, Jochem, Dr., 68165 Mannheim (DE); Kindler, Alois, Dr., 67269 Grünstadt (DE); Knoll, Christian, Dr., 67141 Neuhofen (DE); Mohry, Andre, Dr., 50389 Wesseling (DE)

(56) Entgegenhaltungen:
- EP-A- 1 281 703
- WO-A-03/074502
- JP-B- 45 011 906
- MAEDA, ITSUTOSHI ET AL: "The Synthetic Intermediate of Pyridoxine. II.The Thermal Cyclisation of Ethyl alpha-Isocyanopropionate to 5-Ethoxy-4-methyloxazole" BULL. CHEM. SOC. JAP., Bd. 44, Nr. 5, 1971, XP001105834
- SCHOELLKOPF, ULRICH ET AL: "Synthesis with.alpha.-metalated isocyanides. XXV. Ethyl 4-cyano-2-isocyanoalkanoates, ethyl 4-cyano-2- (formylamino)alkanoates, and ethyl 4-cyano-5(4)-pyrroline-2- carboxylate from.alpha.-metalated ethyl isocyanoalkanoates and acrylonitriles" CHEM. BER. (1973), 106(10), 3382-90 , XP001105793

## Beschreibung

Die vorliegende Erfindung betrifft ein Verfahren zur kontinuierlichen Herstellung von 5-alkoxy-substituierten Oxazolen, insbesondere zur kontinuierlichen Herstellung von 4-Methyl-5-alkoxy-substituierten Oxazolen sowie ein Verfahren zur Herstellung von Pyridoxin-Derivaten.

5-alkoxy-substituierte Oxazole sind wertvolle Synthesbausteine in der organischen Chemie. Als wichtige Vorprodukte für die Synthese und industrielle Produktion von Vitamin B₆ haben 4-Methyl-5-alkoxy-substituierte Oxazole besondere Bedeutung (Turchi et al., Chem. Rev. 1975, 75, 416).

Ein wirtschaftliches und im großen Maßstab durchführbares Verfahren zur Herstellung von 5-alkoxy-substituierten Oxazolen, insbesondere von 4-Methyl-5-alkoxy-substituierten Oxazolen ist daher von hoher Bedeutung.

Es ist bekannt, α-Isocyanälkylsäureester diskontinuierlich durch thermische Isomerisierung in die entsprechenden 5-alkoxy-substituierten Oxazole umzuwandeln.

Itov et al., Khimiko-Farmatsevticheskii Zhurnal, 1978, 12, 102 bis 106 und Mishchenlo et al., Khimiko-Farmatsevticheskii Zhurnal, 1988, 7, 856 bis 860 beschreiben eine diskontinuierliche, thermische Zyklisierung von α-Isocyanpropionsäureester zu den entsprechenden 4-Methyl-5-alkoxy-substituierten Oxazolen bei 135°C. Die durch Verwendung verschiedener Lösungsmittel erreichten Ausbeuten an 4-Methyl-5-alkoxy-substituierten Oxazolen liegen bei 4 bis 36 %. Das Verfahren weist den Nachteil einer geringen Selektivität und damit den Nachteil auf, dass große Mengen an Nebenprodukten entstehen. Häufigste Nebenprodukte dieser Reaktion sind das nicht umgesetzte Edukt (Ausbeute: 33 bis 55 %) sowie der umgelagerte α-Nitrilpropionsäureester (Ausbeute 1 bis 39 %).

Maeda et al., Bull. Chem. Soc. Japan, 1971, 44, 1407 bis 1410 offenbaren eine diskontinuierliche, thermische Zyklisierung von verschiedenen α-Isocyancarbonsäureestern zu den entsprechenden 5-alkoxy-substituierten Oxazolen bei Temperaturen von 150 bis 180°C. Je nach Substituenten werden Ausbeuten von 5,1 bis 28,2 % erreicht.

In JP 54-20493 wird ein diskontinuierliches Verfahren zur Herstellung von 4-Methyl-5-alkoxy-substituierten Oxazolen durch thermische Zyklisierung von α-Isocyanpropionsäureester bei Temperaturen von 155 und 170°C in Gegenwart eines tertiären Amins beschrieben. Dabei werden zwar verbesserte Selektivitäten an den gewünschten Oxazolen erreicht (34 bis 91,5 %) jedoch führt der geringe Umsatz (11,1 bis 49,4 %) noch zu keinen befriedigenden Ausbeuten.

Alle Verfahren des Standes der Technik weisen den Nachteil geringer Umsätze und geringer Selektivitäten und damit geringer Ausbeuten an 5-alkoxy-substituierten Oxazolen auf. Aufgrund der diskontinuierlichen Verfahrensweise weisen die im Stand der Technik bekannten Verfahren nur geringe Raum-Zeit-Ausbeuten auf.

Der Erfindung lag daher die Aufgabe zugrunde, ein weiteres Verfahren zur Herstellung von 5-alkoxy-substituierten Oxazolen mit vorteilhaften Eigenschaften zur Verfügung zu stellen, das die Nachteile des Standes der Technik nicht mehr aufweist und die 5-alkoxy-substituierten Oxazole in hohen Ausbeuten und hohen Raum-Zeit-Ausbeuten liefert.

Dementsprechend wurde ein Verfahren zur kontinuierlichen Herstellung von 5-alkoxy-substituierten Oxazolen der Formel I gefunden, 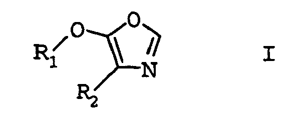 wobei
- R₁: einen, gegebenenfalls substituierten, C₁-C₆-Alkylrest und
- R₂: Wasserstoff oder einen, gegebenenfalls substituierten, C₁-C₆-Alkylrest
bedeuten,
indem man kontinuierlich zugeführte α-Isocyanalkylsäureester der Formel II 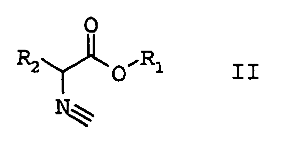 in Gegenwart von kontinuierlich zugeführten Basen
bei Temperaturen größer 80°C
in einem Reaktor in die 5-alkoxy-substituierten Oxazole der Formel I umwandelt und die Reaktionsprodukte kontinuierlich aus dem Reaktor ausführt.

Ein weiterer Gegenstand der vorliegenden Erfindung betrifft ein insérer la page 3a Unter einem gegebenenfalls substituierten, C₁-C6-Alkylrest werden für die Reste R₁ und R₂ unabhängig voneinander verzweigte oder unverzweigte, gegebenenfalls substituierten C₁-C6-Alkylreste verstanden, wie beispielsweise gegebenenfalls substituiertes Methyl, Ethyl, Propyl, 1-Methylethyl, n-Butyl, 1-Methylpropyl, 2-Methylpropyl, 1,1-Dimethylethyl, Pentyl, 1-Methylbutyl, 2-Methylbutyl, 1,2-Dimethylpropyl, 1,1-Dimethylpropyl, 2,2-Dimethylpropyl, 1-Ethylpropyl, Hexyl, 1-Methylpentyl, 1,2-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethyl-butyl, 2-Ethylbutyl.

Die Art der Substituenten ist nicht kritisch. Die C₁-C₆-Alkylreste können je nach freien Bindungsmöglichkeiten bis zu 6 Substituenten enthalten, vorzugsweise ausgewählt aus der Gruppe Aryl, Hydroxyaryl, -NO_{2,} -NH_{2,} -OH, -CN, -COOH, oder Halogen, insbesondere F oder Cl.

In einer bevorzugten Ausführungsform sind die C₁-C₆-Alkylreste der Reste R₁ und R₂ nicht substituiert.

Bevorzugte Reste für R₁ sind C₁-C₄-Alkyl-Reste, wie beispielsweise Methyl, Ethyl, iso-Propyl, n-Propyl, n-Butyl, sec-Butyl oder tert-Butyl, besonders bevorzugt n-Butyl.

Bevorzugte Reste für R₂ sind Wasserstoff und C₁-C₄-Alkyl-Reste, wie beispielsweise Methyl, Ethyl, iso-Propyl, n-Propyl, n-Butyl, sec-Butyl oder tert-Butyl, besonders bevorzugt Methyl.

Bevorzugt ist die Kombination der bevorzugten Reste für R₁ und R₂, besonders bevorzugt ist die Kombination R₁ = n-Butyl und R₂ = Methyl.

In einer besonders bevorzugten Ausführungsform des erfindungsgemäßen Verfahrens wird demnach α-Isocyanpropionsäure-n-butylester in 4-Methyl-5-n-Butoxy-Oxazol umgewandelt.

Verfahren zur kontinuierlichen Herstellung von 5- alkoxy substituieren Oxazolender Formel (1) 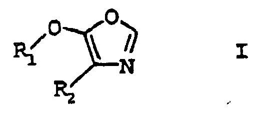 wobei
- R₁: einen, gegebenenfalls substituierten, C₁-C₆-Alkylrest und
- R₂: Wasserstoff oder einen, gegebenenfalls substituierten, C₁-C₆-Alkylrest
bedeuten,
indem man kontinuierlich zugeführte α-Isocyanalkylsäureester der Formel II 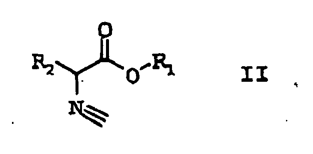 in Gegenwart von kontinuierlich zugeführten Hilfsstoffen
bei Temperaturen größer 80°C
in einem Reaktor in die 5-alkoxy-substituierten oxazole der Formel I umwandelt
und die Reaktionsprodukte kontinuierlich aus dem Reaktor ausführt, wobei man als Reaktor einen Rohrreaktor verwendet.

Die im erfindungsgemäßen Verfahren verwendeten α-Isocyanalkylsäureester der Formel II können in beliebiger Reinheit eingesetzt werden.

Die α-Isocyanalkylsäureester der Formel II können in an sich bekannter Weise aus den entsprechenden Formamidosäureestern der Formel V 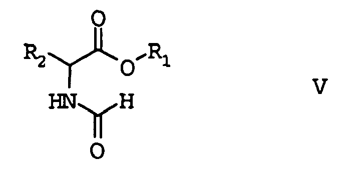 durch Umsetzung mit Phosphoroxychlorid oder Phosgen in Gegenwart von Basen hergestellt werden. Gängige Synthesemethoden sind in Itov et al., Khimiko-Farmatsevticheskii Zhurnal, 1978, 12, 102-106; Maeda et al., Bull. Chem. Soc. Japan, 1971, 44, 1407-1410; Ugi et al., Chem. Ber. 1961, 94, 2814; Chem. Ber. 1960, 93, 239-248, Angew. Chem. 1965, 77, 492-504, Chem. Ber. 1975, 1580-1590, DE 30 29 231 A1 und J. Heterocyclic Chemistry 1988, 17, 705 beschrieben.

Unter Hilfsstoffen werden im erfindungsgemäßen Verfahren chemische Verbindungen verstanden, vorzugsweise chemische Verbindungen die die Cyclisierungsreaktion beschleunigen oder das thermodynamische Gleichgewicht in Richtung des gewünschten Produkts verschieben. Bevorzugte Hilfsstoffe sind Cyclisierungshilfsstoffe, ausgewählt aus aus der Gruppe Basen, Alkohole oder Ester.

Unter Basen werden im erfindungsgemäßen Verfahren Verbindungen mit Brönsted-Basen-Eigenschaften verstanden. Bevorzugte Basen sind tertiäre Amine, wie beispielsweise Triethylamin, Triisopropylamin, Tri-n-Butylamin, Dimethylcyclohexylamin, Tris(2-ethylhexyl)amin, N-Methyl-pyrrolidon, N,N,N'N'-Tetramethyl-1,3-propandiamin, N,N-Diethylanilin oder N,N-Dibutyl-anilin. Besonders bevorzugt ist die Verwendung von Tri-n-butylamin als Base.

Bevorzugte Alkohole sind gegebenenfalls substituierte, C₁-C₆-Alkanole, wie beispielsweise Methanol, Ethanol, n-Propanol, isoPropanol, n-Butanol, sec-Butanol, tert-Butanol, n-Pentanol oder n-Hexanol. Besonders bevorzugt ist die Verwendung von n-Butanol als Alkohol.

Bevorzugte Ester sind gegebenenfalls substituierte C₁-C₆-Alkansäure-C₁-C₆-alkylester, wie beispielsweise Essigsäure-methylester, Essigsäure-ethylester, Essigsäure-propylester, Essigsäuren-butylester, Essigsäure-tert-butylester, Essigsäure-hexylester, Propionsäure-methylester, Propionsäure-ethylester, Propionsäurepropylester, Propionsäure-n-butylester, Propionsäure-tert-butylester, Propionsäure-hexylester, Butansäure-methylester, Butansäure-ethylester, Butansäure-propylester, Butansäure-n-butylester, Butansäure-tert-butylester oder Butansäure-hexylester. Besonders bevorzugt ist die Verwendung von Propionsäure-n-butylester als Ester.

Die Hilfsstoffe können als einzelne Verbindungen oder auch in Form von Mischungen verwendet werden. Bevorzugt werden die Hilfsstoffe als einzelne Verbindungen verwendet.

Unter 80°C findet keine nennenswerte thermische Zyklisierung statt. Die Temperatur bei der erfindungsgemäßen Umwandlung beträgt daher mindestens 80°C.

In einer bevorzugten Ausführungsform erfolgt das erfindungsgemäße Verfahren bei Temperaturen von 100 bis 200°C, besonders bevorzugt bei Temperaturen von 120 bis 170°C, ganz besonders bevorzugt bei Temperaturen von 130 bis 170°C.

Das Molverhältnis von Hilfsstoff zu α-Isocyanalkylsäureester der Formel II ist nicht kritisch und beträgt vorzugsweise 10:1 bis 0,05:1.

Im erfindungsgemäßen Verfahren werden die α-Isocyanalkylsäureester der Formel II und die Hilfsstoffe entweder als Gemisch oder getrennt kontinuierlich einem Reaktor zugeführt, in dem Reaktor die α-Isocyanalkylsäureester der Formel II in die 5-alkoxy-substituierten Oxazole der Formel I umgewandelt und anschließend die Reaktionsprodukte kontinuierlich aus dem Reaktor entfernt.

Als Reaktoren können prinzipiell alle Reaktoren verwendet werden, die eine kontinuierliche Fahrweise ermöglichen.

In einer bevorzugten Ausführungsform des erfindungsgemäßen Verfahrens verwendet man als Reaktor einen Rohrreaktor. Vorzugsweise weist dieser kontinuierlich betriebene Rohrreaktor keine Rückvermischung auf.

Dementsprechend bevorzugt ist ein Rohrreaktor mit einer Bodenstein-Zahl größer gleich 50, wobei die Bodenstein-Zahl in an sich bekannter Weise, beispielsweise wie in Fitzer-Fritz, Technische Chemie, 3.Auflage, Springer-Verlag, Seite 288 f. beschrieben bestimmt werden kann.

Vorzugsweise weist der Rohrreaktor zudem eine theoretische Kesselzahl größer gleich 50 auf.

Der Druck im Rohrreaktor und die Verweilzeit sind nicht kritisch, vorzugsweise wird der Reaktor jedoch druckgeregelt betrieben und die Verweilzeit über die Edukt-Dosierung geregelt.

Der Druck im Rohrreaktor beträgt vorzugsweise mindestens 2 bar, bevorzugter 3 bar bis 9 bar, besonders bevorzugt 4 bis 7 bar, insbesondere 5 bar.

Die Verweilzeit beträgt vorzugsweise 1 bis 8 Stunden, bevorzugter 2 bis 6 Stunden, besonders bevorzugt 3 bis 5 Stunden, insbesondere 4 Stunden.

In einer besonders bevorzugten Ausführungsform dieser Verfahrensvariante unter Verwendung eines Rohrreaktors, erfolgt die Umsetzung der Verbindungen der Formel II im Rohrreaktor vorteilhäfterweise nur bis zu einem Teilumsatz. Der Teilumsatz beträgt vorzugsweise 40 bis 70 %, bevorzugter 50 bis 60 %, insbesondere 54 %.

In dieser bevorzugten Ausführungsform unter Verwendung eines Rohrreaktors ist es besonderes vorteilhaft, den Austrag des Rohrreaktors in eine kontinuierlich betriebene Kolonne einzuspeisen und in der Kolonne kontinuierlich destillativ in eine leichtsiedende Fraktion enthaltend die Verbindungen der Formel I und in eine schwersiedende Fraktion, enthaltend nicht umgesetzte Verbindungen der Formel II und Hilfsstoffe aufzutrennen.

Bevorzugte Kolonnen sind dabei die nachstehend beschriebenen, kontinuierlich betriebenen Reaktionskolonnen.

Ein weiterer Umsatz kann bei dieser Ausführungsform mit nachgeschalteter Kolonne in der nachgeschalteten Kolonne erfolgen, wie nachstehend beschrieben.

In einer weiter bevorzugten Ausführungsform dieser Variante unter Verwendung eines Rohrreaktors mit nachgeschalteter Kolonne, wird die aus der Kolonne ausgetragene schwersiedende Fraktion, enthaltend nicht umgesetzte Verbindungen der Formel II und Hilfsstoffe in die Umsetzung zurückführt. Dadurch wird über den Gesamtprozess ein Umsatz der Verbindungen der Formel II von 100 % erreicht.

Eine andere bevorzugten Ausführungsform des kontinuierlichen, erfindungsgemäßen Verfahrens lässt sich besonders vorteilhaft durchführen, indem man bei der Umwandlung im Reaktor, also gleichzeitig mit der Umwandlung die 5-alkoxy-substituierten Oxazole der Formel I aus dem Reaktionsgemisch abtrennt. Diese Abtrennung erfolgt bevorzugt ebenfalls kontinuierlich.

Es gibt viele Ausgestaltungen von Reaktoren, die für diese bevorzugte Ausführungsform des erfindungsgemäßen Verfahrens in Frage kommen. Bevorzugte Reaktoren sollten die Eigenschaft aufweisen, eine kontinuierliche Umwandlung bei gleichzeitiger Abtrennung eines Reaktionsprodukts ermöglichen.

Beispielsweise können als Reaktoren Blasen mit aufgesetzter Kolonne, Extraktionskolonnen, Glockenbodenkolonnen, Membranreaktoren, Lord-Reaktoren oder Reaktionskolonnen verwendet werden.

Wie dem Fachmann bekannt, werden unter dem Begriff Kolonne, wenn nicht anders erwähnt, ein Kolonnenaufbau mit Sumpf verstanden.

Unter einer aufgesetzten Kolonne wird dementsprechend nur der Kolonnenaufbau ohne Sumpf verstanden.

Unter Reaktionskolonnen werden vorzugsweise Kolonnen verstanden, deren Einbauten einen hold-up aufweisen, also beispielsweise Kolonnen mit Böden, Schüttungen, Packungen oder Füllkörpern.

In einer besonders bevorzugten Ausführungsform des erfindungsgemäßen Verfahrens erfolgt die Umsetzung in einer Reaktionskolonne als Reaktor.

Die Reaktionskolonnen können von der Bauart und den Einbauten beliebig ausgestaltet sein. Besonders bevorzugt ist die Verwendung einer Trennwandkolonne als Reaktionskolonne.

Eine Reaktionskolonne, die auf verschiedenste Arten ausgestaltet sein kann, hat die Eigenschaft als Reaktor gleichzeitig eine Umwandlung von Reaktanten und die Abtrennung durch Rektifikation der 5-alkoxy-substituierten Oxazole der Formel I aus dem Reaktionsgemisch zu ermöglichen.

In dieser bevorzugten Ausführungsform unter Verwendung einer Reaktionskolonne ist es weiterhin von Vorteil die Rektifikationsparameter so einzustellen, dass
- A: die Umwandlung der α-Isocyanalkylsäureester der Formel II in die 5-alkoxy-substituierten Oxazole der Formel I auf den Einbauten und gegebenenfalls im Sumpf der Reaktionskolonne erfolgt,
- B: die bei der Umwandlung entstehenden 5-alkoxy-substituierten Oxazole der Formel I kontinuierlich mit dem Kopfstrom oder Seitenstrom der Reaktionskolonne abgetrennt werden und
- C: der Hilfsstoff, sowie die bei der Umwandlung gegebenenfalls entstehenden Hochsieder kontinuierlich und unabhängig voneinander mit dem Sumpfstrom oder Seitenstrom der Reaktionskolonne abgetrennt werden.

Je nach Ausgestaltungsform der Reaktionskolonne und der verwendeten Reaktanten, wird dies durch unterschiedliche Einstellungen der Rektifikationsparameter erreicht. Geeignete Rektifikationsparameter sind beispielsweise Temperatur, Druck, Rücklaufverhältnis in der Kolonne, Ausgestaltung der Kolonne und deren Einbauten, Wärmeführung und Verweilzeit, insbesondere im Sumpf oder Energieeintrag, die der Fachmann durch Routineversuche so optimieren kann, dass die Merkmal A, B und C erreicht werden.

In Merkmal C können die Hilfsstoffe insbesondere auch getrennt von den Hochsiedern in einem zweiten Seitenstrom abgetrennt werden.

Unter Seitenstrom wird erfindungsgemäß der kontinuierliche Austrag einer Substanz über einen Seitenabzug der Kolonne verstanden.

Im erfindungsgemäßen Verfahren wird der Druck am Kolonnenkopf so eingestellt, dass die Temperatur im Sumpf und auf den Einbauten mindestens 80°C, vorzugsweise 100 bis 200°C, besonders bevorzugt 120 bis 170°C beträgt.

Typischerweise wird der Kopfdruck der Kolonne auf 5 bis 800 mbar eingestellt, so dass sich der daraus je nach verwendeter Kolonnenart und gegebenenfalls verwendeter Kolonneneinbauarten ergebende Sumpfdruck typischerweise 5 mbar bis atmosphärischer Druck beträgt.

Die Verweilzeit in der Reaktionskolonne beträgt typischerweise zwischen 10 Minuten und 7 Stunden, vorzugsweise zwischen 30 Minuten und 4 Stunden.

Es ist möglich, dass die 5-alkoxy-substituierten Oxazole der Formel I mit den verwendeten Hilfsstoffen ein azeotropes Gemisch bilden, so dass die 5-alkoxy-substituierten Oxazole der Formel I über den Kopfstrom als azeotropes Gemisch abgetrennt werden.

In diesem Fall ist es vorteilhaft, den Kopfdruck und damit auch automatisch den Sumpfdruck in der Kolonne, je nach hergestelltem 5-alkoxy-substituierten Oxazol der Formel I und verwendetem Hilfsstoff so einzustellen, dass der Anteil an Hilfsstoff im Azeotrop im Kopfstrom möglichst gering ist.

Die Abtrennung des Hilfsstoffs aus dem Kopfstrom-Azeotrop erfolgt in diesem Fall in an sich bekannter Weise, beispielsweise durch eine sich anschließende zweite Rektifikation unter Verwendung eines anderen Drucks (Zweidruck-Destillation).

Beispielsweise bildet das nach dem erfindungsgemäßen Verfahren hergestellte 4-Methyl-5-n-Butoxy-Oxazol mit der Base Tri-n-Butylamin ein Azeotrop. Bei Einstellung eines Kopfdrucks von 100 mbar setzt sich das Azeotrop am Kopfstrom aus 91 Gew.-% 4-Methyl-5-n-Butoxy-Oxazol und 9 Gew.-% Tri-n-Butylamin zusammen.

Die Abtrennung von Tri-n-Butylamin aus dem Kopfstrom-Azeotrop kann in diesem Fall beispielsweise durch eine sich anschließende zweite Rektifikation bei einem Kopfdruck von 10 mbar erfolgen.

Das erfindungsgemäße Verfahren kann in Anwesenheit oder Abwesenheit von Lösungsmitteln durchgeführt werden. In einer bevorzugten Ausführungsform erfolgt das erfindungsgemäße Verfahren ohne Lösungsmittel.

In einer weiteren bevorzugten Ausführungsform erfolgt das erfindungsgemäße Verfahren in Gegenwart eines inerten Lösungsmittels. Unter einem inerten Lösungsmittel werden vorzugsweise unpolare und polare aprotische Lösungsmittel wie Toluol, Xylol oder Chlorbenzol, Dichlormethan, Dichlorethan, Dichlorbenzol, Ethylencarbonat, Propylencarbonat, insbesondere Chlorbenzol verstanden.

Im Falle der Verwendung eines Lösungsmittels kann das Lösungsmittel beispielsweise mit dem Hilfsstoff und den α-Isocyanalkylsäureester der Formel II im Gemisch oder jede einzelne Komponente separat kontinuierlich der Kolonne zugeführt werden.

Im Falle der Verwendung eines inerten Lösungsmittels im erfindungsgemäßen Verfahren werden die Rektifikationsparameter vorzugsweise so einstellt, dass
- A: die Umwandlung der α-Isocyanalkylsäureester der Formel II in die 5-alkoxy-substituierten Oxazole der Formel I auf den Einbauten und gegebenenfalls im Sumpf der Reaktionskolonne erfolgt,
- B1: für den Fall, dass das Lösungsmittel einen höheren Siedepunkt als die bei der Umwandlung entstehenden 5-alkoxy-substituierten Oxazole der Formel I aufweist, die 5-alkoxy-substituierten Oxazole der Formel I kontinuierlich mit dem Kopfstrom und das Lösungsmittel kontinuierlich über den Seitenstrom oder Sumpfstrom der Reaktionskolonne abgetrennt werden,
- B2: für den Fall, dass das Lösungsmittel einen niedrigeren Siedepunkt als die die bei der Umwandlung entstehenden 5-alkoxysubstituierten Oxazole der Formel I aufweist, die 5-alkoxysubstituierten Oxazole der Formel I kontinuierlich mit einem Seitenstrom und das Lösungsmittel kontinuierlich mit dem Kopfstrom der Reaktionskolonne abgetrennt wird und
- C: der Hilfsstoff, sowie die bei der Umwandlung gegebenenfalls entstehenden Hochsieder kontinuierlich und unabhängig voneinander mit dem Sumpfstrom oder Seitenstrom der Reaktionskolonne abgetrennt werden.

Als Einbauten der Reaktionskolonne können jegliche Ausführungsformen verwendet werden, wie beispielsweise Kolonnenböden, Schüttungen, Füllkörper oder strukturierte Packungen.

Besonders vorteilhafte Kolonnenböden ermöglichen eine hohe Verweilzeit der Flüssigkeit, wobei die Verweilzeit auf den Einbauten der Reaktionskolonne bevorzugt mindestens 30 min beträgt.

Bevorzugte Kolonnenböden sind beispielsweise Ventilböden, bevorzugt Glockenböden oder verwandte Bauarten wie zum Beispiel Tunnelböden, Lord-Reaktoren und andere Einbauten oder Thormannböden.

Bevorzugte strukturierte Packungen sind beispielsweise strukturierte Packungen vom Typ Mellapack^{®} (Fa. Sulzer), BY^{®} (Fa. Sulzer), B1^{®} (Fa. Montz) oder A3^{®} (Fa. Montz) oder Packungen mit vergleichbaren Ausgestaltungen.

Das erfindungsgemäße Verfahren weist gegenüber dem Stand der Technik folgende Vorteile auf:

Mit dem erfindungsgemäßen Verfahren werden Selektivitäten von über 95 % bezogen auf den eingesetzten α-Isocyanalkylsäureester der Formel II erreicht.

Der Umsatz liegt bei nahezu 100 %, so dass die Ausbeute an 5-alkoxy-substituierten Oxazolen der Formel I über 95 % bezogen auf den eingesetzten α-Isocyanalkylsäureester der Formel II betragen.

Ein weiterer Vorteil des Verfahrens ist die kontinuierliche Fahrweise. Die Raumzeitausbeute ist deutlich größer als bei den bisher bekannten Verfahren.

Das erfindungsgemäße Verfahren stellt einen neuen und vorteilhaften Teilsyntheseschritt im Verfahren zur Herstellung von Pyridoxin-Derivaten der Formel IX dar, 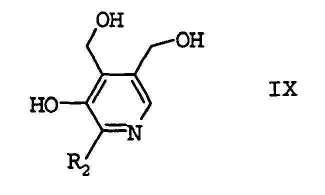 insbesondere zur Herstellung von Pyridoxin (Vitamin B₆; Formel IX, R₂ = Methyl).

Daher betrifft die Erfindung ferner ein Verfahren zur Herstellung von Pyridoxin-Derivaten der Formel IX
in dem man Aminosäuren der Formel III 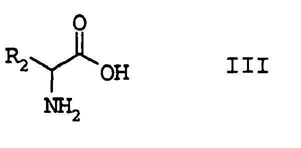 in Aminosäureester der Formel IV umwandelt, 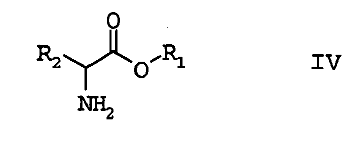 diese in Formamidosäureester der Formel V umwandelt, 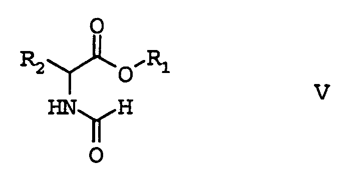 diese in α-Isocyanalkylsäureester der Formel II umwandelt, 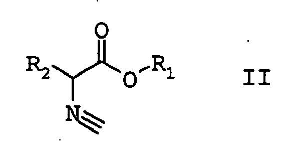 diese erfindungsgemäß in einem kontinuierlichen Verfahrensschritt in Gegenwart von Basen
bei Temperaturen größer 80°C
in 5-alkoxy-substituierten Oxazole der Formel I umwandelt, 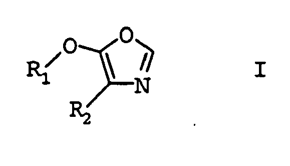 diese mit geschützten Diolen der Formel VI, 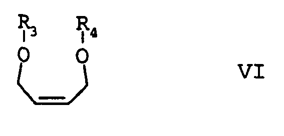 wobei
- R_{3,}R₄: unabhängig voneiunander oder R₃ und R₄ zusammen eine Schutzgruppe der Hydroxyfunktion
bedeuten,
zu den Diels-Alder-Addukten der Formel VII umsetzt, 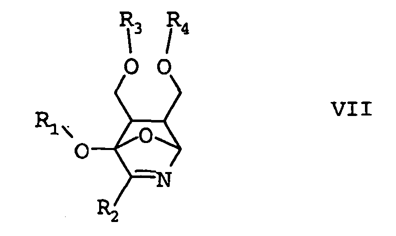 und diese durch saure Behandlung und Abspaltung der Schutzgruppe in die Pyridoxin-Derivate der Formel IX überführt.

Das Gesamtverfahren ist bis auf den erfindungsgemäßen neuen, vorteilhaften Teilschritt der kontinuierlichen Umwandlung von α-Isocyanalkylsäureester der Formel II in 5-alkoxy-substituierten Oxazole der Formel aus Ullmann's Encyclopedia of Industrial Chemistry 1996, Vol. A 27, Seite 533 bis 537 bekannt.

Ausgangsstoffe für die Gesamtsynthese sind wohlfeile Aminosäuren der Formel III, vorzugsweise Alanin (R₂ = Methyl). Diese werden in an sich bekannter Weise, beispielsweise durch säurekatalysierte Veresterung mit den Alkoholen R₁-OH, vorzugsweise n-Butanol in Aminosäureester der Formel IV umgewandelt. Diese Veresterung kann aber auch durch andere Methoden, wie beispielsweise durch Aktivierung der Säurefunktion und basenkatalysierte Veresterung erreicht werden. Weiter Methoden sind in US 3,227,721 beschrieben.

Die Aminosäureester der Formel IV werden in an sich bekannter Weise, beispielsweise wie in US 3,227,721 beschrieben in die Formamidosäureester der Formel V umgewandelt.

Die Formamidosäureester der Formel V werden anschließend in an sich bekannter Weise, wie vorstehend beschrieben in die α-Isocyanalkylsäureester der Formel II umgewandelt.

Die α-Isocyanalkylsäureester der Formel II werden, wie vorstehend beschrieben mit dem erfindungsgemäßen Verfahren kontinuierlich in die 5-alkoxy-substituierten Oxazole der Formel I umgewandelt.

Im bevorzugten Gesamtverfahren wird dieser Teilschritt in den bevorzugten Ausführungsformen, wie vorstehend beschrieben, durchgeführt.

Die 5-alkoxy-substituierten Oxazole der Formel I werden anschließend mit geschützten Diolen der Formel VI zu den Diels-Alder-Addukten der Formel VII umgesetzt.

Dieser Teilschritt kann dem erfindungsgemäßen Verfahren nachgeschaltet sein, kann aber auch durch kontinuierliche Zuführung der geschützten Diole der Formel VI zum Reaktor des erfindungsgemäßen Verfahrens gleichzeitig mit der Umwandlung der α-Isocyanalkylsäureester der Formel II in die 5-alkoxy-substituierten Oxazole der Formel I erfolgen. Die Zuführung erfolgt dabei entweder in Mischung mit den α-Isocyanalkylsäureester der Formel II, dem Hilfsstoff und gegebenenfalls dem Lösungsmittel oder als separate Komponente. Als Produkt werden in diesem Fall die 5-alkoxy-substituierten Oxazole direkt in Form ihres Diels-Alder-Addukts über den Sumpfaustrag der Kolonne entfernt.

Unter den Resten R_{3,} R₄ werden unabhängig voneiunander eine Schutzgruppe, vorzugsweise eine säurelabile Schutzgruppe der Hydroxyfunktion verstanden.

Prinzipiell kann jede säurelabile Schutzgruppe verwendet werden. Bevorzugte säurelabile Schutzgruppen sind die in der Literatur bekannten säurelabilen Schutzgruppen für Hydroxygruppen (T.W. Greene, Protective Groups in Organic Synthesis, John Wiley & Sons New York, 1981,Seite 14-71; P.J. Kocienski, Protecting Groups, Georg Thieme Verlag Stuttgart, 1994, Seite 21-94).

Ferner können, in einer bevorzugten Ausführungsform, die Reste R₃ und R₄ gemeinsam eine säurelabile Schutzgruppe der beiden Hydroxyfunktionen bilden. Vorzugsweise bilden dazu die beiden Hydroxyfunktionen mit Ketonen oder Aldehyden, wie beispielsweise Aceton oder Isobutyraldehyd ein cyclisches Acetal.

Durch anschließende saure Behandlung der Diels-Alder-Addukte der Formel VII erfolgt unter Abspaltung des Alkohols R₁-OH die Aromatisierung zum Pyridoxingerüst. Die Abspaltung der säurelabilen Schutzgruppe(n), die in der Regel durch saure, wässerige Behandlung erfolgt liefert die Pyridoxin-Derivaten der Formel IX, insbesondere Pyridoxin (Vitamin B6, R₂ = Methyl).

Der Alkohol R₁-OH und die Schutzgruppen R₃ und R₄ können wiedergewonnen und im Gesamtverfahren wieder eingesetzt werden.

Die Verwendung des erfindungsgemäßen neuen vorteilhaften Teilschritts im Gesamtverfahren führt zu einer Steigerung der Gesamtausbeute.

Die nachstehenden Beispiele erläutern die Erfindung

### Beispiel 1

### Kontinuierliche Herstellung von 4-Methyl-5-n-Butoxy-Oxazol in einer Trennwandkolonne

In eine kontinuierlich betriebene Trennwandkolonne (4,8 m x 64 mm) gefüllt mit 3 x 3 mm V₂A-Raschigringen und einer Trennwand von 2,4 m Höhe mit 60 theoretischen Trennstufen wurde ein Gemisch aus 20,5 Gew.-% α-Isocyanpropionsäure-n-butylester (R₁= n-Butyl, R₂ = Methyl) und 79,5 Gew.-% Tri-n-butylamin eingeleitet.

Bei 500 mbar Kopfdruck und einer Sumpftemperatur von 165°C geht 4-Methyl-5-n-Butoxy-Oxazol als Azeotrop mit Tri-n-butylamin (90:10 Gew.-%) mit einem Siedepunkt von 158°C über Kopf. Im Kolonnensumpf werden Hochsieder und Tributylamin abgezogen. Der Umsatz betrug 98,4 %, die Selektivität 99 %. Die Ausbeute an 4-Methyl-5-n-Butoxy-Oxazol betrug 95 % bezogen auf den eingesetzten α-Isocyanpropionsäure-n-butylester.

Das Azeotrop wurde anschließend in der gleichen Kolonne bei einem Kopfdruck von 10 mbar getrennt. Man erhält als Kopfprodukt ein Azeotrop mit der Zusammensetzung 4-Methyl-5-n-Butoxy-Oxazol:Tri-n-butylamin = 70:30 und im Seitenabzug reines 4-Methyl-5-n-Butoxy-Oxazol mit einem Siedepunkt von 98°C. Die Destillationsausbeute betrug 99 % (40 % reines 4-Methyl-5-n-Butoxy-Oxazol und 60 % 4-Methyl-5-n-Butoxy-Oxazol als Azeotrop, das in die erste Destillation zurückgeführt wurde). Das Rein-4-Methyl-5-n-Butoxy-Oxazol hatte einen Gehalt von 99,8 %.

### Beispiel 2

### Kontinuierliche Herstellung von 4-Methyl-5-n-Butoxy-Oxazol in einer Trennwandkolonne mit Lösungsmittel

In eine kontinuierlich betriebenen Trennwandkolonne (4,8 m x 64 mm) gefüllt mit 3 x 3 mm V₂A-Raschigringen und einer Trennwand von 2,4 m Höhe mit 60 theoretischen Trennstufen wurde ein Gemisch aus 13,1 Gew.-% α-Isocyanpropionsäure-n-butylester (R₁= n-Butyl, R₂ = Methyl), 32,2 Gew.-% Monochlorbenzol und 50,1 Gew.-% Tri-n-butylamin eingeleitet.

Bei 300 mbar Kopfdruck und einer Sumpftemperatur von 169°C geht mit einem Siedepunkt von 90°C Monochlorbenzol über Kopf, im Seitenabzug wird mit einer Übergangstemperatur von 151°C 4-Methyl-5-n-Butoxy-Oxazol als Azeotrop mit Tri-n-butylamin (88:12 Gew.-%) erhalten. Im Kolonnensumpf werden Hochsieder und Tributylamin abgezogen. Der Umsatz betrug 99,5 % die Selektivität 99 %. Die Ausbeute an 4-Methyl-5-n-Butoxy-Oxazol betrug 94 % bezogen auf den eingesetzten α-Isocyanpropionsäure-n-butylester.

Das Azeotrop wurde analog Beispiel 1 getrennt.

### Beispiel 3

### Kontinuierliche Herstellung von 4-Methyl-5-n-Butoxy-Oxazol in einer Reaktionskolonne mit Lösungsmittel

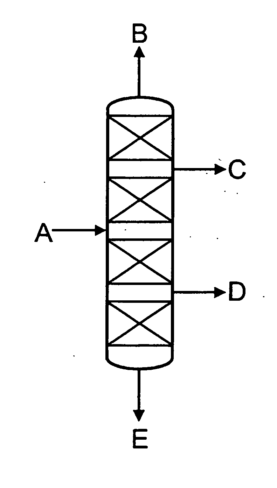

In einer Kolonne gemäß Beispiel 1 jedoch ohne Trennwand (siehe Abbildung 1) wurde über den Zulauf (A) ein Gemisch aus 20,6 Gew.-% Chlorbenzol, 5,2 Gew.-% α-Isocyanpropionsäuren-butylester (R₁= n-Butyl, R₂ = Methyl) und 72,60 Gew.-% Tris(2-ethylhexyl)amin kontinuierlich zugefahren.

Bei einem Kopfdruck von 300 mbar und einer Sumpftemperatur von 165°C geht das Lösungsmittel über Kopfabzug (B). Das 4-Methyl-5-n-Butoxy-Oxazol wird über den Seitenabzug (C) in einer Ausbeute von 99 % erhalten. Das Amin wird über den Sumpfaustrag (E) ausgeschleust.

### Beispiel 4

### Kontinuierliche Herstellung von 4-Methyl-5-n-Butoxy-Oxazol in einer Reaktionskolonne

Gemäß Beispiel 3 wird über den Zulauf (A) ein Gemisch aus 13,14 % α-Isocyanpropionsäure-n-butylester und 86,86 % Tris(2-ethylhexyl)amin kontinuierlich zugefahren.

Bei einem Kopfdruck von 400 mbar und einer Sumpftemperatur von 165°C wird das 4-Methyl-5-n-Butoxy-Oxazol über den Kopfabzug (B) ausgetragen und das Amin über den Sumpfaustrag (E) ausgeschleust. Die Ausbeute an 4-Methyl-5-n-Butoxy-Oxazol beträgt 98,8 %.

### Beispiel 5

### Kontinuierliche Herstellung von 4-Methyl-5-iso-Butoxy-Oxazol in einer Reaktionskolonne

Gemäß Beispiel 3 wird über den Zulauf (A) ein Gemisch aus 22,7 % α-Isocyanpropionsäure-iso-butylester und 77,3 % N,N-Dibutylanilin kontinuierlich zugefahren.

Bei einem Kopfdruck von 300 mbar und einer Sumpftemperatur von 160°C wird das 4-Methyl-5-iso-Butoxy-Oxazol bei einer Temperatur von 150°C über den Kopfabzug (B) ausgetragen. Das Amin wird über den Seitenabzug D bei 161°C erhalten. Die Ausbeute an 4-Methyl-5-iso-Butoxy-Oxazol beträgt 91 %.

### Beispiel 6

### Kontinuierliche Herstellung von 4-Methyl-5-n-Butoxy-Oxazol in einer Reaktionskolonne

Gemäß Beispiel 5 wird über den Zulauf (A) ein Gemisch aus 11,8 % α-Isocyanpropionsäure-n-butylester und 88,2 % N,N-Dibutylanilin kontinuierlich zugefahren. Man erhält 4-Methyl-5-n-Butoxy-Oxazol mit einer Ausbeute von 98,7 % über den Kopfabzug B und das Amin über den Seitenabzug D.

### Beispiel 7

### Kontinuierliche Herstellung von 4-Methyl-5-butyloxy-oxazol (MOX) in einer Reaktionskolonne unter Verwendung von n-Butanol als Cyclisierungshilfsstoff

In eine kontinuierlich betriebenen Trennwandkolonne (4,8 m x 64 mm) gefüllt mit 3 x 3 mm V₂A-Raschigringen und einer Trennwand von 2,4 m Höhe mit 60 theoretischen Trennstufen wurde ein Gemisch aus 30 Gew.-% α-Isocyan-propionsäure-n-butylester und 70 Gew.-% n-Butanol über den Zulauf (A) eingeleitet.

Bei einem Kopfdruck von 300 mbar und einer Sumpftemperatur von 165°C wird n-Butanol über Kopf abgetrennt. Der Kolonnensumpf wird mit Phthalsäure-di-n-butylester als Zwischensieder auf einem kontinuierlichen Stand gehalten.

Im Seitenabzug wird 4-Methyl-5-butyloxy-oxazol (MOX) als 97 % Reinstoff mit einer Ausbeute von 94 % erhalten.

### Beispiel 8

### Kontinuierliche Herstellung von 4-Methyl-5-butyloxy-oxazol (MOX) in einer Reaktionskolonne unter Verwendung von Propionsäure-n-butylester als Cyclisierungshilfsstoff

Die Apparatur und die experimentelle Durchführung entspricht Beispiel 7.

Analog zu Beispiel 7 wird ein Gemisch aus 35 Gew.-% α-Isocyanpropionsäure-*n*-butylester und 65 Gew.-% Propionsäure-n-butylester kontinuierlich eingeleitet.

Bei einem Kopfdruck von 300 mbar und einer Sumpftemperatur von 165°C wird Propionsäure-n-butylester über Kopf abgetrennt. Der Kolonnensumpf wird mit Phthalsäure-di-n-butylester als Zwischensieder als Zwischensieder auf einem kontinuierlichen Stand gehalten.

Im Seitenabzug wird 4-Methyl-5-butyloxy-oxazol (MOX) als 95 % Reinstoff mit einer Ausbeute von 92 % erhalten.

### Beispiel 9

### Kontinuierliche Herstellung von 4-Methyl-5-butyloxy-oxazol (MOX) in einem Rohrreaktor und anschließender Reindestillation in einer kontinuierlich betriebenen Kolonne (Hilfsstoff tri-n-Butylamin (TBA))

Die Apparatur bestand aus einem 100 ml Rohrreaktor mit einer theoretischen Kesselzahl von 120. Der Reaktor wurde druckgeregelt bei 5 bar betrieben. Der Zulauf erfolgte mengengeregelt, so dass eine Verweilzeit von 4 h erreicht wurde.

Als Zulauf wurde α-Isocyan-propionsäure*-n*-butylester (ICE) und tri-n-Butylamin (TBA) im molaren Verhältnis von 1/3 (mol ICE /mol TBA) gemischt.

Der Austrag wurde aufgefangen. Es wurde bei einem Umsatz von 54 % ICE bei einer Selektivität von 92 % eine MOX-Ausbeute von 50 % erhalten.

Der Reaktoraustrag wurde gesammelt und in einer kontinuierlich betriebenen Kolonne ohne Trennwand (gemäß Beispiel 3) destilliert.

Über den Zulauf (Stutzen A) wurde die Lösung zugefahren. Bei einem Druck von 50 mbar und einer Sumpf temperatur von 80 bis 90°C wurde das Gemisch MOX/Amin über Kopf ausgetragen und ICE/Amin über einen Seitenabzug (gasförmig) eine Trennstufe über dem Sumpf entnommen. Der Sumpf enthielt die hochsiedenden Nebenkomponenten. Er wurde ausgetragen und verworfen.

Das ICE/Amin-Gemisch wurde in die Reaktion zurückgeführt, der MOX-Kopfaustrag zu reinem MOX aufgereinigt.

Über den Gesamtprozess wurde ein ICE-Umsatz von 100 % erreicht. Die Selektivität bezüglich MOX betrug 90 %. Der Rest sind Hochsieder, die als Sumpfprodukt der kontinuierlich betriebenen Destillation ausgeschleust wurden.

### Beispiel 10

### Kontinuierliche Herstellung von 4-Methyl-5-butyloxy-oxazol (MOX) in einem Rohrreaktor und anschließender Reindestillation in einer kontinuierlich betriebenen Kolonne (Hilfsstoff Di-Ethyl-phenyl-amin)

In einer Apparatur gemäß Beispiel 9 erfolgte die gleiche Umsetzung unter Verwendung von Di-Ethyl-phenyl-amin als Hilfsstoff.

Der Austrag des Rohrreaktors wurde aufgefangen. Es wurde bei einem Umsatz von 58 % ICE bei einer Selektivität von 90 % eine MOX-Ausbeute von 53 % erhalten.

Über den Gesamtprozess wurde ein ICE- Umsatz von 100 % erreicht. Die Selektivität bezüglich MOX betrug 87 %. Der Rest waren Hochsieder, die als Sumpfprodukt der kontinuierlich betriebenen Destillation ausgeschleust wurden.

### Beispiel 11

### Kontinuierliche Herstellung von 4-Methyl-5-butyloxy-oxazol (MOX) in einem Rohrreaktor und anschließender Reindestillation in einer kontinuierlich betriebenen Kolonne (Hilfsstoff n-Butanol)

In einer Apparatur gemäß Beispiel 9 erfolgt die gleiche Umsetzung unter Verwendung von n-Butanol als Hilfsstoff.

Der Austrag des Rohrreaktors wurde aufgefangen. Es wurde bei einem Umsatz von 54 % ICE bei einer Selektivität von 93 % eine MOX-Ausbeute von 50 % erhalten.

Die Kolonne wurde in diesem Fall mit Trennwand bei 500 mbar Vakuum betrieben. Über Kopf wurde n-Butanol abgenommen. Im Seitenabzug wurde MOX mit einer Reinheit von 98 % erhalten. Über Sumpf wurde ICE mit einer Reinheit von 95 % erhalten.

Der Sumpf wurde mit kontinuierlich dosiertem Di-Butyl-phthalat (5 Masse-% bezogen auf den Zulauf) auf einem konstanten Stand gehalten. Der Überlauf enthielt die Hochsieder und wurde entsogt.

Über den Gesamtprozess wurde ein ICE- Umsatz von 100 % erreicht. Die Selektivität bezüglich MOX betrug 89 %. Der Rest waren Hochsieder, die als Sumpfprodukt der kontinuierlich betriebenen Destillation ausgeschleust wurden.

## Patentansprüche

1. Verfahren zur kontinuierlichen Herstellung von 5-alkoxy-substituierten Oxazolen der Formel I, 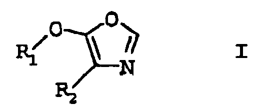 wobei
R₁ einen, gegebenenfalls substituierten, C₁-C₆-Alkylrest und
R₂ Wasserstoff oder einen, gegebenenfalls substituierten, C₁-C₅-Alkylrest
bedeuten,
indem man kontinuierlich zugeführte α-Isocyanalkylsäureester der Formel II 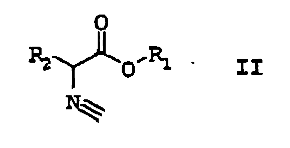 in Gegenwart von kontinuierlich zugeführten Basen
bei Temperaturen größer 80°C
in einem Reaktor in die 5-alkoxy-substituierten Oxazole der Formel I umwandelt
und die Reaktionsprodukte kontinuierlich aus dem Reaktor ausführt.

2. Verfahren zur kontinuierlichen Herstellung von 5-alkoxy-substituierten Oxazolen der Formel I, 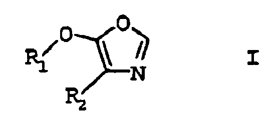 wobei
R₁ einen, gegebenenfalls substituierten, C₁-C₆-Alkylrest und
R₂ Wasserstoff oder einen, gegebenenfalls substituierten, C₁-C₆-Alkylrest
bedeuten,
indem man kontinuierlich zugeführte α-Isocyanalkylsäureester der Formel II 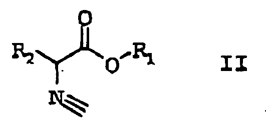 in Gegenwart von kontinuierlich zugeführten Hilfsstoffen
bei Temperaturen größer 80°C
in einem Reaktor in die 5-alkoxy-substituierten Oxazole der Formel I umwandelt
und die Reaktionsprodukte kontinuierlich aus dem Reaktor ausführt, wobei man als Reaktor einen Rohrreaktor verwendet.

3. Verfahren nach Anspruch 2, **dadurch gekennzeichnet dass** man als Hilfsstoffe Cyclisierungshilfsstoffe, ausgewählt aus der Gruppe Basen, Alkohole oder Ester verwendet.

4. Verfahren nach Anspruch 2 oder 3, **dadurch gekennzeichnet, dass** der Rohrreaktor eine Bodenstein-Zahl größer gleich 50 aufweist.

5. Verfahren nach einem der Ansprüche 2 bis 4, **dadurch gekennzeichnet, dass** der Rohrreaktor eine theoretische Kesselzahl größer gleich 50 aufweist.

6. Verfahren nach einem der Ansprüche 2 bis 5, **dadurch gekennzeichnet, dass** man den Austrag des Rohrreaktors in eine kontinuierlich betriebene Kolonne einspeist und in der Kolonne kontinuierlich destillativ in eine leichtsiedende Fraktion enthaltend die Verbindungen der Formel I und in eine schwersiedende Fraktion, enthaltend nicht umgesetzte Verbindungen der Formel II und Hilfsstoffe auftrennt.

7. Verfahren nach Anspruch 6, **dadurch gekennzeichnet, dass** man die schwersiedende Fraktion, enthaltend nicht umgesetzte Verbindungen der Formel II und Hilfsstoffe in die Umsetzung zurückführt.

8. Verfahren nach Anspruch 1, **dadurch gekennzeichnet, dass** man gleichzeitig mit der Umwandlung die 5-alkoxy-substituierten Oxazole der Formel I aus dem Reaktionsgemisch abtrennt.

9. Verfahren nach Anspruch 8, **dadurch gekennzeichnet, dass** man als Reaktor eine Reaktionskolonne verwendet und gleichzeitig mit der Umwandlung die 5-alkoxy-substituierten Oxazole der Formel I durch Rektifikation aus dem Reaktionsgemisch abtrennt

10. Verfahren nach Anspruch 9, **dadurch gekennzeichnet, dass** man die Rektifikationsparameter so einstellt, dass
A die Umwandlung der α-Isocyanalkylsäureester der Formel II in die 5-alkoxy-substituierten Oxazole der Formel I auf den Einbauten und gegebenenfalls im Sumpf der Reaktions-kolonne erfolgt,
B die bei der Umwandlung entstehenden 5-alkoxy-substituierten Oxazole der Formel I kontinuierlich mit dem Kopfstrom oder Seitenstrom der Reaktionskolonne abgetrennt werden und
C der Hilfsstoff, sowie die bei der Umwandlung gegebenen-falls entstehenden Hochsieder kontinuierlich und unabhängig voneinander mit dem Sumpfstrom oder Seitenstrom der Reaktionskolonne abgetrennt werden.

11. Verfahren nach einem der Ansprüche 9 oder 10, **dadurch gekennzeichnet, dass** man die Umwandlung in Gegenwart eines inerten Lösungsmittels durchführt und die Reaktionsparameter so einstellt, dass
A die Umwandlung der α-Isocyanalkylsäureester der Formel II in die 5-alkoxy-substituierten Oxazole der Formel I auf den Einbauten und gegebenenfalls im Sumpf der Reaktionskolonne erfolgt,
B1 für den Fall, dass das Lösungsmittel einen höheren Siedepunkt als die bei der Umwandlung entstehenden 5-alkoxy-substituierten Oxazole der Formel I aufweist, die 5-alkoxy-substituierten Oxazole der Formel I kontinuierlich mit dem Kopfstrom und das Lösungsmittel kontinuierlich über den Seitenstrom oder Sumpfstrom der Reaktionskolonne abgetrennt werden,
B2 für den Fall, dass das Lösungsmittel einen niedrigeren Siedepunkt als die die bei der Umwandlung entstehenden 5-alkoxy-substituierten Oxazole der Formel I aufweist, die 5-alkoxy-substituierten Oxazole der Formel I kontinuierlich mit einem Seitenstrom und das Lösungsmittel kontinuierlich mit dem Kopfstrom der Reaktionskolonne abgetrennt wird und
C der Hilfsstoff, sowie die bei der Umwandlung gegebenen-falls entstehenden Hochsieder kontinuierlich und unabhängig voneinander mit dem Sumpfstrom oder Seitenstrom der Reaktionskolonne abgetrennt werden.

12. Verfahren nach einem der Ansprüche 9 bis 11, **dadurch gekennzeichnet, dass** man als Reaktionskolonne eine Trennwandkolonne verwendet.

13. Verfahren nach einem der Ansprüche 9 bis 12, **dadurch gekennzeichnet, dass** man, für den Fall dass der Hilfsstoff mit den 5-alkoxy-substituierten Oxazolen der Formel I ein Azeotrop bildet, den Kopfdruck in der Kolonne, so einstellt, dass der Anteil an Hilfsstoff im Azeotrop im Kopfstrom möglichst gering ist.

14. Verfahren nach einem der Ansprüche 9 bis 13, **dadurch gekennzeichnet, dass** man den Kopfdruck der Kolonne auf 5 bis 800 mbar einstellt und der sich daraus je nach verwendeter Kolonnenart und gegebenenfalls verwendeter Kolonneneinbautenart ergebende Sumpfdruck 10 mbar bis atmosphärischer Druck beträgt.

15. Verfahren zur Herstellung von Pyridoxin-Derivaten der Formel IX 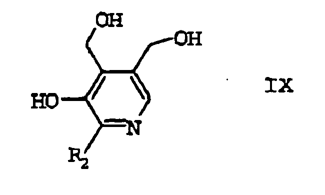 wobei
R₂ Wasserstoff oder einen, gegebenenfalls substituierten, C₁-C₆-Alkylrest bedeutet,
in dem man Aminosäuren der Formel III 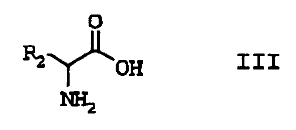 in Aminosäureester der Formel IV umwandelt, 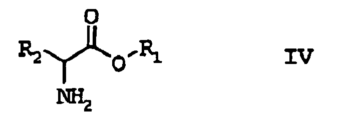 wobei
R₁ einen, gegebenenfalls substituierten, C₁-C₆-Alkylrest bedeutet,
diese in Formamidosäureester der Formel V umwandelt, 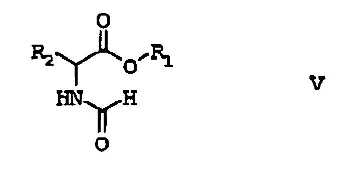 diese in α-Isocyanalkylsäureester der Formel II umwandelt, 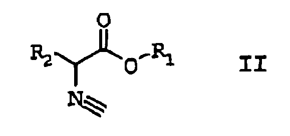 diese in einem kontinuierlichen Verfahrensschritt gemäß einem verfahren nach Anspruch 1
in Gegenwart von Basen
bei Temperaturen größer 80°C
in 5-alkoxy-substituierten Oxazole der Formel I umwandelt, 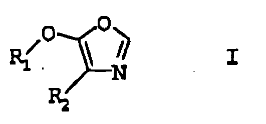 diese mit geschützten Diolen der Formel VI, 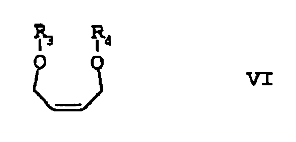 wobei
R_{3,} R₄ unabhängig voneiunander oder R₃ und R₄ zusammen eine Schutzgruppe der Hydroxyfunktion
bedeuten,
zu den Diels-Alder-Addukten der Formel VII umsetzt, 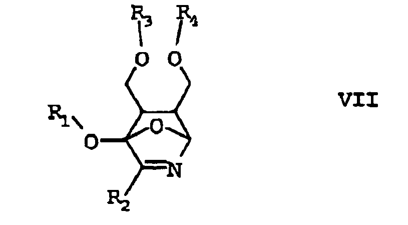 und diese durch saure Behandlung und Abspaltung der Schutzgruppe in die Pyridoxin-Derivaten der Formel IX überführt.

## Claims

1. A process for the continuous preparation of 5-alkoxy-substituted oxazoles of the formula I, 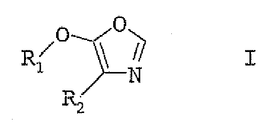 where
R₁ is an optionally substituted C₁-C₆-alkyl radical and
R₂ is hydrogen or an optionally substituted C₁-C₆-alkyl radical,
by converting α-isocyanoalkanoic esters of the formula II 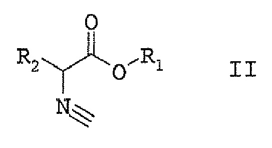 which are fed in continuously,
in the presence of bases which are fed in continuously, at temperatures above 80°C
in a reactor into the 5-alkoxy-substituted oxazoles of the formula I
and discharging the reaction products continuously from the reactor.

2. A process for the continuous preparation of 5-alkoxy-substituted oxazoles of the formula I, 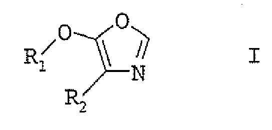 where
R₁ is an optionally substituted C₁-C₆-alkyl radical and
R₂ is hydrogen or an optionally substituted C₁-C₆-alkyl radical,
by converting α-isocyanoalkanoic esters of the formula II 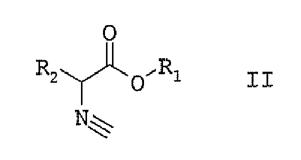 which are fed in continuously,
in the presence of assistants which are fed in continuously, at temperatures above 80°C
in a reactor into the 5-alkoxy-substituted oxazoles of the formula I
and discharging the reaction products continuously from the reactor, the reactor used being a tubular reactor.

3. The process according to claim 2, wherein assistants used comprise cyclization assistants selected from the group consisting of bases, alcohols and esters.

4. The process according to claim 2 or 3, wherein the tubular reactor has a Bodenstein number greater than or equal to 50.

5. The process according to any one of claims 2 to 4, wherein the tubular reactor has a theoretical CSTR count greater than or equal to 50.

6. The process according to any one of claims 2 to 5, wherein the effluent from the tubular reactor is fed into a continuously operated column and is continuously separated in the column by distillation into a low-boiling fraction comprising the compounds of the formula I and a high-boiling fraction comprising unconverted compounds of the formula II and assistants.

7. The process according to claim 6, wherein the high-boiling fraction comprising unconverted compounds of the formula II and assistants is recycled into the reaction.

8. The process process according to claim 1, wherein the 5-alkoxy-substituted oxazoles of the formula I are removed from the reaction mixture simultaneously with the conversion.

9. The process according to claim 8, wherein a reaction column is used as reactor, and the 5-alkoxy-substituted oxazoles of the formula I are removed from the reaction mixture by rectification simultaneously with the conversion.

10. The process according to claim 9, wherein the rectification parameters are adjusted so that
A the conversion of the α-isocyanoalkanoic esters of the formula II into the 5-alkoxy-substituted oxazoles of the formula I takes place on the internals and, if appropriate in the bottom of the reaction column,
B the 5-alkoxy-substituted oxazoles of the formula I produced in the conversion are removed continuously with the overhead stream or side stream of the reaction column and
C the assistant, and the high boilers produced if appropriate in the conversion, are removed continuously and independently of one another with the bottom stream or side stream of the reaction column.

11. The process according to either of claims 9 and 10, wherein the conversion is carried out in the presence of an inert solvent, and the reaction parameters are adjusted so that
A the conversion of the α-isocyanoalkanoic esters of the formula II into the 5-alkoxy-substituted oxazoles of the formula I takes place on the internals and, if appropriate in the bottom of the reaction column,
B1 in the case where the solvent has a higher boiling point than the 5-alkoxy-substituted oxazoles of the formula I produced in the conversion,
the 5-alkoxy-substituted oxazoles of the formula I are removed continuously with the overhead stream, and the solvent is removed continuously via the side stream or bottom stream of the reaction column, and
B2 in the case where the solvent has a lower boiling point than the 5-alkoxy-substituted oxazoles of the formula I produced in the conversion, the 5-alkoxy-substituted oxazoles of the formula I are removed continuously with a side stream, and the solvent is removed continuously with the overhead stream of the reaction column, and
C the assistant, and the high boilers produced if appropriate in the conversion, are removed continuously and independently of one another with the bottom stream or side stream of the reaction column.

12. The process according to any of claims 9 to 11, wherein a dividing wall column is used as reaction column.

13. The process according to any of claims 9 to 12, wherein in the case where the assistant forms an azeotrope with the 5-alkoxy-substituted oxazoles of the formula I, the overhead pressure in the column is adjusted so that the proportion of assistant in the azeotrope in the overhead stream is minimized.

14. The process according to any of claims 9 to 13, wherein the overhead pressure of the column is adjusted to 5 to 800 mbar, and the bottom pressure which results therefrom, depending on the type of column used and, if appropriate, the type of column internals used, is 10 mbar to atmospheric pressure.

15. A process for preparing pyridoxine derivatives of the formula IX 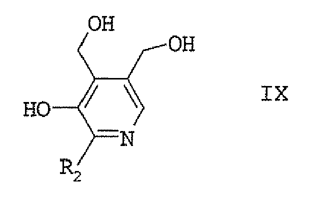 where
R₂ is hydrogen or an optionally substituted C₁-C₆-alkyl radical,
by converting amino acids of the formula III 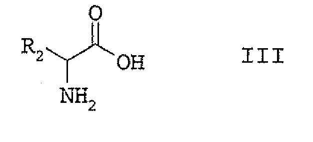 into amino acid esters of the formula IV, 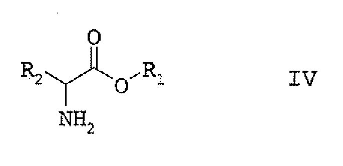 where
R₁ is an optionally substituted C₁-C₆-alkyl radical,
converting the latter into formamido acid esters of the formula V, 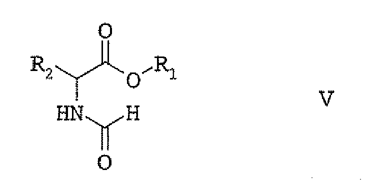 converting the latter into α-isocyanoalkanoic esters of the formula II 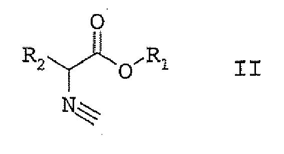 converting the latter in a continuous process step according to a process according to claim 1
in the presence of bases
at temperatures above 80°C
into 5-alkoxy-substituted oxazoles of the formula I, 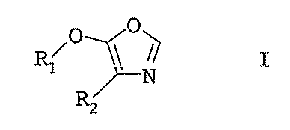 reacting the latter with protected diols of the formula VI, 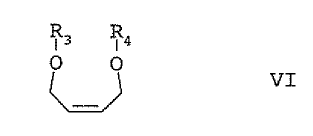 where
R₃, R₄ are, independently of one another, or R₃ and R₄ together, are a protective group of the hydroxyl function,
to give the Diels-Alder adducts of the formula VII 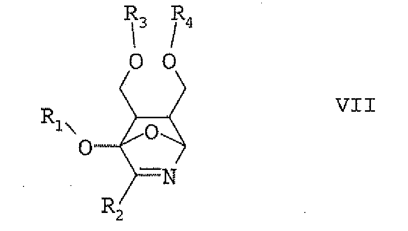 and converting the latter by acid treatment and elimination of the protective group into the pyridoxine derivatives of the formula IX.

## Revendications

1. Procédé de fabrication en continu d'oxazoles substitués par un 5-alcoxy de formule 1 : 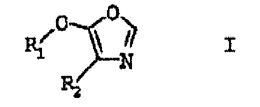 dans laquelle :
R₁ signifie un radical alkyle en C₁-C₆ éventuellement substitué et
R₂ signifie un hydrogène ou un radical alkyle en C₁-C₆ éventuellement substitué,
en transformant en continu les esters d'acide α-isocyanalkylique amenés de formule II : 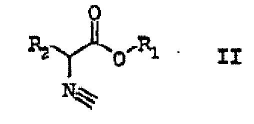 en présence de bases amenées en continu
à des températures supérieures à 80°C,
dans un réacteur en les oxazoles substitués par un 5-alcoxy de formule I
et en évacuant les produits réactionnels en continu hors du réacteur.

2. Procédé de fabrication en continu d'oxazoles substitués par un 5-alcoxy de formule I : 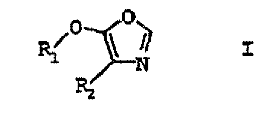 dans laquelle :
R₁ signifie un radical alkyle en C₁-C₆ éventuellement substitué et
R₂ signifie un hydrogène ou un radical alkyle en C₁-C₆ éventuellement substitué,
en transformant en continu les esters d'acide α-isocyanalkylique amenés de formule II : 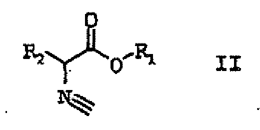 en présence d'adjuvants amenés en continu
à des températures supérieures à 80°C,
dans un réacteur en les oxazoles substitués par un 5-alcoxy de formule I
et en évacuant hors du réacteur en continu les produits réactionnels, et on utilise comme réacteur un réacteur tubulaire.

3. Procédé selon la revendication 2, **caractérisé en ce qu'**on utilise comme adjuvants des adjuvants de cyclisation sélectionnés parmi le groupe des bases, des alcools ou des esters.

4. Procédé selon la revendication 2 ou 3, **caractérisé en ce que** le réacteur tubulaire présente un nombre de Bodenstein plus grand ou égal à 50.

5. Procédé selon l'une des revendications 2 à 4, **caractérisé en ce que** le réacteur tubulaire présente un nombre de cuves théorique plus grand ou égal à 50.

6. Procédé selon l'une des revendications 2 à 5, **caractérisé en ce qu'**on introduit la sortie du réacteur tubulaire dans une colonne fonctionnant en continu et on sépare, dans la colonne, en continu et par distillation en une fraction de produits à bas point d'ébullition contenant les composés de formule 1 et en une fraction de produits à point d'ébullition élevé contenant les composés non réagi de formule II ainsi que les adjuvants.

7. Procédé selon la revendication 6, **caractérisé en ce qu'**on recycle la fraction de produits à point d'ébullition élevé contenant les composés non réagi de formule II et les adjuvants dans la réaction.

8. Procédé selon la revendication 1, **caractérisé en ce qu'**on sépare, simultanément avec la transformation, les oxazoles substitués par un 5-alcoxy de formule I hors du mélange réactionnel.

9. Procédé selon la revendication 8, **caractérisé en ce qu'**on utilise comme réacteur une colonne de réaction et qu'on sépare simultanément avec la transformation les oxazoles substitués par un 5-alcoxy de formule I par rectification hors du mélange réactionnel.

10. Procédé selon la revendication 9, **caractérisé en ce qu'**on règle les paramètres de rectification de telle sorte que :
A la transformation des esters d'acide α-isocyanalkylique de formule II en les oxazoles substitués par un 5-alcoxy de formule I se fait sur les éléments encastrés et éventuellement au fond de la colonne de réaction
B les oxazoles substitués par un 5-alcoxy produits pendant la transformation et de formule I sont séparés en continu avec le courant de tête ou le courant latéral de la colonne de réaction, et
C l'adjuvant, ainsi que les produits à point d'ébullition élevé éventuellement produits pendant la transformation sont séparés en continu et indépendamment les uns des autres avec le courant de fond ou le courant latéral de la colonne de réaction.

11. Procédé selon l'une des revendications 9 ou 10, **caractérisé en ce qu'**on opère la transformation en présence d'un solvant inerte et qu'on règle les paramètres réactionnels de telle manière que
A la transformation des esters d'acide α-isocyanalkylique de formule II en les oxazoles substitués par un 5-alcoxy et de formule I s'effectue sur les éléments encastrés et éventuellement au fond de la colonne de réaction,
B1 pour le cas où le solvant présente un point d'ébullition plus élevé que les oxazoles substitués par un 5-alcoxy produits pendant la transformation et de formule I, les oxazoles substitués par un 5-alcoxy de formule I sont séparés en continu avec le courant de tête et le solvant est separé, en continu, par le courant latéral ou le courant de fond de la colonne de réaction,
B2 pour le cas où le solvant présente un point d'ébullition plus bas que celui des oxazoles substitués par un 5-alcoxy produits pendant la transformation et de formule I, les oxazoles substitués par un 5-alcoxy de formule I sont séparés en continu avec un courant latéral et le solvant l'est en continu avec le courant de tête de la colonne de réaction, et
C l'adjuvant, ainsi que les produits à point d'ébullition élevé éventuellement produits pendant la transformation sont séparés en continu et indépendamment les uns des autres avec le courant de fond ou le courant latéral de la colonne de réaction.

12. Procédé selon l'une des revendications 9 à 11, **caractérisé en ce qu'**on utilise comme colonne de réaction une colonne cloisonnée.

13. Procédé selon l'une des revendications 9 à 12, **caractérisé en ce qu'**on règle, dans le cas où l'adjuvant avec les oxazoles substitués par un 5-alcoxy de formule I forment un azéotrope, la pression de tête de la colonne de telle manière que la proportion en adjuvant dans l'azéotrope dans le courant de tête est la plus faible possible.

14. Procédé selon l'une des revendications 9 à 13, **caractérisé en ce qu'**on règle la pression de tête de la colonne entre 5 et 800 mbars et que la pression du fond de colonne qui en résulte, en fonction du type de colonne utilisée et éventuellement du type d'éléments encastrés de colonne utilisés, va de 10 mbars à la pression atmosphérique.

15. Procédé de fabrication de dérivés de pyridoxine de formule IX : 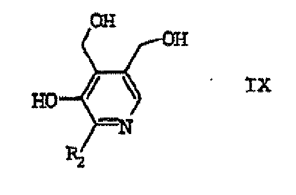 dans laquelle :
R₂ signifie un hydrogène ou un radical alkyle en C₁-C₆ éventuellement substitué,
en transformant des acides aminés de formule III : 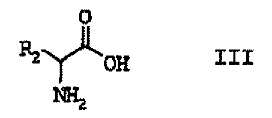 en esters d'acide aminé de formule IV : 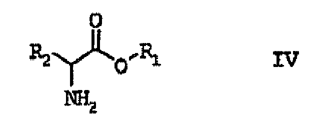 dans laquelle :
R₁ signifie un radical alkyle en C₁-C₆ éventuellement substitué,
on transforme ceux-ci en esters d'acide formamido de formule V : 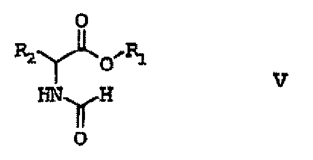 on transforme ceux-ci en esters d'acide α-isocyanalkylique de formule II : 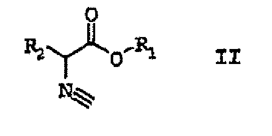 on transforme ceux-ci lors d'une étape de procédé en continu selon un procédé selon la revendication 1
en présence de bases
à des températures supérieures à 80°C
en oxazoles substitués par un 5-alcoxy de formule I : 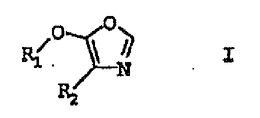 on fait réagir ceux-ci avec des diols protégés de formule VI : 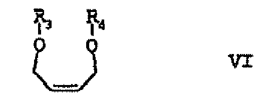 dans laquelle :
R₃, R₄ signifient indépendamment l'un de l'autre ou R₃ et R₄ signifient ensemble un groupe protecteur de la fonction hydroxy
pour obtenir les produits d'addition de Diels-Adler de formule VII : 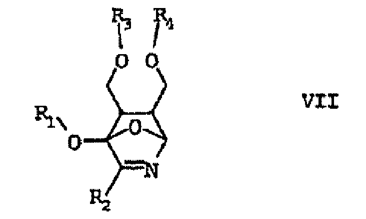 et on transforme ceux-ci par traitement acide et fission du groupe protecteur en les dérivés de pyridoxine de formule IX.
